(19) Europäisches Patentamt
European Patent Office
Office européen des brevets

(11) Publication number: **0 096 013**
**B1**

(12) **EUROPEAN PATENT SPECIFICATION**

(45) Date of publication of patent specification: **23.07.86**

(21) Application number: **83830098.6**

(22) Date of filing: **18.05.83**

(51) Int. Cl.$^4$: **C 07 H 17/08,** A 61 K 31/70, C 07 C 149/23

# (54) Salts of erythromycin showing a combined therapeutic activity, process for their preparation and pharmaceutical formulations containing them.

(30) Priority: **28.05.82 IT 4853982**

(43) Date of publication of application: **07.12.83 Bulletin 83/49**

(45) Publication of the grant of the patent: **23.07.86 Bulletin 86/30**

(84) Designated Contracting States: **AT BE CH DE FR GB LI LU NL SE**

(56) References cited:
**EP-A-0 035 770**
**EP-A-0 052 909**
**FR-A-2 427 821**

**Farmaco, Ed. Prat., Vol. 33(a), pages 379-391, 1978**

**The file contains technical information submitted after the application was filed and not included in this specification**

(73) Proprietor: **Nuovo Consorzio Sanitario Nazionale del Dott. Paolo Malizia**
**Via Svetonio 6**
**I-00136 Roma (IT)**

(72) Inventor: **Casini, Giovanni, Prof**
**Via Caldarola 45/62i**
**I-70100 Bari (IT)**

(74) Representative: **Bazzichelli, Alfredo et al**
**c/o Società Italiana Brevetti Piazza Poli 42**
**I-00187 Roma (IT)**

EP 0 096 013 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to novel compounds formed by a chemical bond of ionic type (true salts) obtained from erythromycin A of the propionic ester (2'-propanoate) of erythromycin A with two acidic compounds endowed with different therapeutic activities; namely, acetylsalicylic acid (o-acetoxybenzoic acid) possessing antipyretic and antiinflammatory activity, and DL-alpha-mercaptopropionylglycine (tiopronine) possessing mucolytic and hepato-protective activity; for the latter acid, salts have been obtained in both diastereomeric forms.

In these compounds the pharmacological activity of the antibiotic moiety and that of the acidic moiety are thus simultaneously present, and since the latter activity may be defined as adjuvant with respect to the activity of the antibiotic, these novel compounds have the advantage of joining two different activities in a single molecule, both contributing to a more complete therapeutic result.

Thus the antipyretic activity of acetylsalicylic acid present as anion in the salts with both antibiotics contributes in lowering fever, while the antibiotic cation contributes to the same effect by eliminating infection that causes fever. In the particular case of rheumatic disease, for which salicylic acid therapy is indicated, the antibiotic can potentiate antirheumatic and antiinflammatory activity of the acidic moiety by counteracting the streptococcal infective origin of the disease.

DL-alpha-mercaptopropionylglycine (hereinafter tiopronine) is an aminoacid derivative containing a mercapto group (—SH); it has been employed therapeutically as a hepatoprotective and as mucolytic agent. In this case therapeutic advantage is obtained particularly for lung diseases, since the mucolytic activity of tiopronine present as anion in the salts with both antibiotics contributes to therapeutic result by making bronchial secretions more fluid, while the antibiotic cation contributes by eliminating infection that causes the disease. Moreover, the hepatoprotective activity of the acidic moiety will protect liver tissue against toxic effects of erythromycin and its ester, the latter observation being of value also for infections of different districts of the organism.

In EP—A—0035770 simple associations are reported of antibiotic substances (among which erythromycin and its propanoate are cited) with adjuvant compounds (among which salicylic acid and its sodium salt is cited), said associations resulting in enhanced oral absorption of the antibiotic. The above cited associations, however, are not chemical compounds in which the two moieties are linked, as are the salts of the present invention and, moreover, acetylsalicylic acid is not cited among the adjuvants.

A report has been published (N. Kikuchi, Jpn. J. Antibiotics, *33* 117, 1980; CA *93*, 38279f) assessing that the activity of antibiotics such as erythromycin is inhibited by mercapto derivatives such as cysteine and its analogs; however, said inhibition arises at different ratios with the mercapto derivatives than those (1:1 in moles) existing in the salts of tiopronine of the invention and it has been indeed verified (EP—A 0057489) that the analogous salts of erythromycin and of its propanoate with N-acetyl-L-cysteine retain all the antimicrobial potency of the original antibiotics. It has been now surprisingly found that the salts of the present invention not only maintain the antimicrobial potency of the original antibiotics, but appear to enhance the therapeutic activity thereof, at least in some cases.

Another report (P. Pomarelli, Farmaco, ed. pr., *33* 379, 1978; CA *90*, 650) shows the therapeutic utility of a simple association between tiopronine and erythromycin: however in contrast to the present salts a chemical compound is not formed between the two moieties in this association. EP—A 0052909 relates to salts of the thioester of tiopronine with p-isobutylphenylpropionic acid with erythromycin and its propanoate; however, in these salts the mercapto group is not free as it is in our tiopronine salts, which is necessary for the mucolytic and hepato-protective activity.

Object of the invention are derivatives of erythromycin A having the general formula:

$$[X^+ \; Y^-]$$

wherein $X^+$ is a cation from the macrolide antibiotic, of formula:

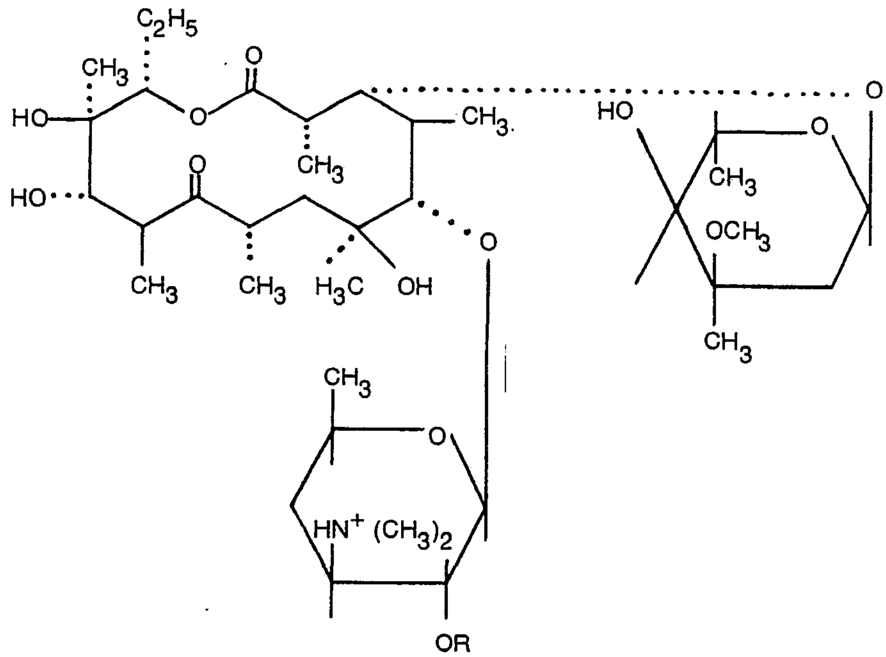

in which R is H or —CO—$CH_2$—$CH_3$ and wherein $Y^-$ is the anion of

A) Acetylsalicylic acid of formula

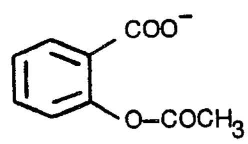

or

B) DL-alpha-mercaptopropionyl-glycine or one of the two enantiomeric forms thereof of formula

$CH_3$—CH—CO—NH—$CH_2$—$COO^-$
|
SH

as well as a process for a preparation thereof and pharmaceutical formulations containing them.

*Preparation of the salts of the invention*

The salts of the present invention are prepared by dissolving the base and acid in stoichiometric amounts in suitable solvents, and allowing the mixture to crystallize at or below room temperature.

The following examples are reported only for illustrative purpose, and show some procedures for preparing salts described in this invention.

Example 1

Propionylerythromycin acetylsalicylate.

To 8.1 g of propionylerythromycin hydrate dissolved in 100 ml of ethyl acetate are added 1.8 g of acetylsalicylic acid dissolved in 20 ml of ethyl acetate; the mixture is allowed to stand at 20°C for one hour and then left at 0°C overnight; a crystalline solid precipitates, which is filtered, washed with solvent and dried at room temperature to give 9.0 g, yield 91%, of propionylerythromycin acetylsalicylate, $C_{40}H_{71}NO_{14}.C_9H_8O_4$.

| | | | |
|---|---|---|---|
| Calculated: | C%: 60.66 | H%: 8.21 | N%: 1.44 |
| Found: | 60.49 | 8.33 | 1.51 |

white crystalline powder M.P. 128°C;
$[\alpha]_D^{20} = -77°$ (c = 1, ethanol);
soluble in 300 p. water and in 4 p. ethanol 95% v/v;
IR spectrum; 3460 1375 1605 1460 1375 1170 1055 1010 750 $cm^{-1}$.

Aqueous solutions acidified or basified and extracted with ethyl acetate, after removal of the solvent give 18.6% of acetylsalicylic acid and 81.4% propionylerythromycin respectively.

In a similar way are prepared:

Erythromycin acetylsalicylate, yield 80%, $C_{36}H_{67}NO_{13}.C_9H_8O_4$.

| | | | |
|---|---|---|---|
| Calculated: | C%: 60.44 | H%: 8.27 | N%: 1.53 |
| Found: | 60.27 | 8.60 | 1.48 |

white crystalline powder, M.P. 130°C;
$[\alpha]_D^{20} = -63°$ (c = 1, ethanol);
soluble in 100 p. water and in 2 p. ethanol 95% v/v;
IR spectrum: 3480 1730 1590 1460 1375 1170 1055 1005 760 $cm^{-}$.

Desalifying as above gives 19.7% of acetylsalicylic acid and 80.3% of erythromycin respectively.

Erythromycin (+)-alpha-mercaptopropionylglycinate, yield 50%, $C_{37}H_{67}NO_{13}.C_5H_9NO_3S.H_2O$.

| | | | |
|---|---|---|---|
| Calculated: | C%: 55.12 | H%: 8.59 | N%: 3.06 |
| Found: | 55.12 | 8.50 | 2.77 |

white crystalline power M.P. 143°C;
$[\alpha]_D^{20} = -57°$ (c = 1, ethanol);
soluble in 25 p. water and in 3 p. ethanol;
IR spectrum: 3420 1725 1650 1600 1380 1165 1080 1010 $cm^{-1}$.

Basifying and extracting as above gives 80.2% of erythromycin; the acid isolable from the salt shows dextrorotatory power; mercapto group titration 1.09 meq $I_2$ per gram.

Propionylerythromycin (+)-alpha-mercaptopropionylglycinate, yield 45%, $C_{40}H_{71}NO_{14}.C_5H_9NO_3S.H_2O$.

| | | | |
|---|---|---|---|
| Calculated: | C%: 55.65 | H%: 8.51 | N%: 2.88 |
| Found: | 55.62 | 8.50 | 2.87 |

white crystalline powder, M.P. 120°C;
$[\alpha]_D^{20} = -60°$ (c = 1 ethanol);
soluble in 90p. water and in 2 p. ethanol 95% v/v;
IR spectrum: 3400 1735 1650 1375 1170 1055 990 $cm^{-1}$.

Desalifying as above gives 81.3% of propionylerythromycin base; the acid isolable from the salt shows dextrorotatory power; mercapto group titration 1.03 meq $I_2$ per gram.

## Example 2

Diastereomeric forms of erythromycin A alpha-mercaptopropionylglycinate.

15.0 g of eythromycin are dissolved in 60 ml of ethyl acetate; 6.5 g (2 moles per mole) of DL-alpha-mercaptopropionylglycine (tiopronine) are dissolved in 10 ml of hot 1-propanol; the solutions are combined and seeded, then left at 0°C overnight; a crystalline solid precipitates, which is filtered, washed with solvent and dried at room temperature to give 17.5 g (95% yield) of erythromycin (+)-alpha-mercaptopropionylglycinate, properties as above. The mother liquors are evaporated *in vacuo* to less than 10 ml volume, and a further 15.0 g of erythromycin dissolved in 60 ml of ethyl acetate are added; leaving at 0°C overnight gives a new crystalline solid which is filtered, washed and dried to give 13.8 g (75% yield) of erythromycin (−)-alpha-mercaptopropionylglycinate $C_{37}H_{67}NO_{13}.C_5H_9NO_3S.H_2O$.

| | | | |
|---|---|---|---|
| Calculated: | C%: 55.12 | H%: 8.59 | N%: 3.06 |
| Found: | 55.07 | 8.48 | 2.83 |

M.P. 121°C; soluble in 15 p. water and 2 p. ethanol 95% v/v;
IR spectrum 3415 1715 1655 1380 1170 1075 1010 $cm^{-1}$;
$[\alpha]_D^{20} = -67°$ (c = 1, ethanol); the acid isolable from the salt has levorotatory power; other analytical properties as diastereomeric salt.

In a similar way are prepared:

Propionylerythromycin (+)-alpha-mercaptopropionylglycinate, yield 90%, properties as above; and propionylerythromycin (−)-alpha-propionylglycinate $C_{40}H_{71}NO_{14}.C_5H_9NO_3S.H_2O$.

| | | | |
|---|---|---|---|
| Calculated: | C%: 55.65 | H%: 8.51 | N%: 2.88 |
| Found: | 55.55 | 8.47 | 2.78 |

M.P. 105°C; $[\alpha]_D^{20} = -80°$; (c = 1, ethanol);
soluble in 60 p. water and in 2 p. ethanol 95% v/v;
I.R. spectrum: 3405 1720 1655 1380 1170 1065 1000 $cm^{-1}$;
the acid isolable from the salt has levorotatory power;
other analytical properties as diastereomeric salt.

*Pharmacological activity*

The salts of the present invention have been subjected to *in vitro* tests for antibacterial activity; they generally show activities of the same order of magnitude as parent antibiotics; particularly interesting is the activity against some hospital strains of Streptococcus beta-haemolyticus which is sometimes greater than that of the original antibiotic, a shown in the following table 1.

TABLE 1

| MIC μg/ml | Streptococcus beta-haemolyticus strain | | | |
|---|---|---|---|---|
| | A | B | C | D |
| Erythromycin | 5 | 1.25 | 0.6 | 0.6 |
| Erythromycin acetylsalicylate | 1.25 | 0.6 | 0.3 | 0.075 |
| Erythromycin (+)-alpha-mercaptopropionylglycinate | 5 | 0.3 | 0.3 | 0.3 |
| Propionylerythromycin | 2.5 | 0.6 | 0.6 | 0.6 |
| Propionylerythromycin acetylsalicylate | 5 | 0.3 | 0.3 | 0.3 |
| Propionylerythromycin (+)-alpha-mercaptopropionylglycinate | 5 | 0.3 | 0.3 | 0.3 |

The pharmacological properties of the acidic moieties will be retained since, as demonstrated by the analytical results, the structural features from which they depend, i.e. the aryl ester bond of acetylsalicylic acid and the mercapto group of alpha-mercaptopropionylglycine are still present.

An additional advantage of the present invention is that the salts are also in general more watersoluble than the corresponding bases, thus permitting aqueous solutions to be employed in therapy.

*Pharmaceutical formulations*

The salts of the present invention can be used as active ingredients in various solid (capsules, tablets) and liquid (suspensions, syrups) formulations usual for oral administration.

Moreover, their solubility in water allows their use by parenteral injection, using a freeze-dried product to be dissolved just before use.

It is also possible to use such aqueous solutions as formulations for aerosol inhalation: for these formulations, and also for pediatric oral formulations, salts of propionylerythromycin are preferred, since they are not endowed with a bitter taste.

Topical use of the same active ingredients in ointments is also possible.

Examples of pharmaceutical formulations are given only for illustrative purpose:

Propionylerythromycin acetylsalicylate:

— Oral capsule, 300 mg each.

— Granulate for oral suspension: 50 mg/ml, with usual excipients, sweeteners and flavoring agents.

— Ampules for injection: 250 mg in freeze-dried form plus ampules with 5 ml bidistilled non-pyrogenic water as solvent.

— Suppositories: 300 mg, with suitable excipients.

Propionylerythromycin (+)-alpha-mercaptopropionylglycinate:

— Granulate for oral suspension: 50 mg/ml, formulated as above.

— Ampules for aerosol: 500 mg in freeze-dried form plus ampules with 5 ml bidistilled sterile water as solvent.

— Suppositories: 300 mg, with suitable excipients.

For clinical use the active ingredients of the present invention can be administered at a dosage of 1.2 to 2.4 g/day, preferably administered four times a day.

**Claims**

1. Derivatives of erythromycin A having the general formula: $[X^+ Y^-]$, wherein $X^+$ is a cation from the macrolide antibiotic of formula:

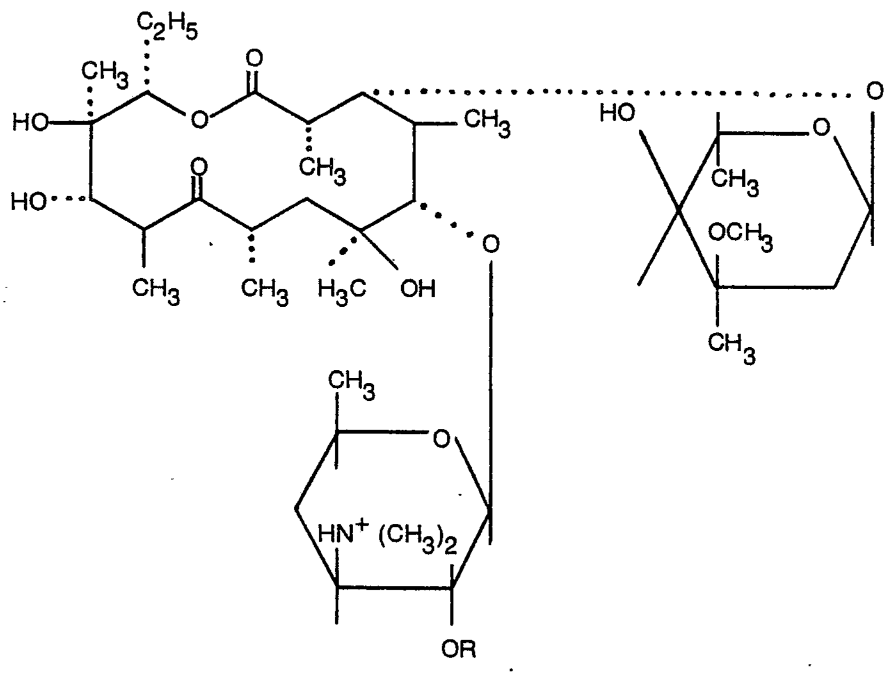

in which R is H or $—CO—CH_2—CH_3$ and wherein $Y^-$ is the anion of

A) Acetylsalicylic acid of formula:

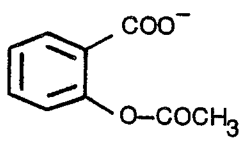

or B) DL-alpha-mercaptopropionyl-glycine or one of the two enantiomeric forms thereof, of formula:

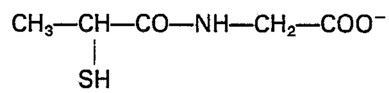

2. Erythromycin salt with acetylsalicylic acid, according to claim 1.

3. Propionylerythromycin salt with acetylsalicylic acid, according to claim 1.

4. Erythromycin salt with DL-alpha-mercaptopropionylglycine, according to claim 1.

5. Propionylerythromycin salt with DL-alpha-mercaptopropionylglycine according to claim 1.

6. A diastereomeric form of erythromycin salt with alpha-mercaptopropionyl-glycine, according to claim 4, derived from one of the two enantiomeric forms of alpha-mercaptopropionylglycine.

7. A diastereomeric form of propionylerythromycin salt with alpha-mercaptopropionylglycine, according to claim 5, derived from one of the two enantiomeric forms of alpha-mercaptopropionylglycine.

8. A process for the preparation of the compounds as claimed in claims 1 to 5 comprising reacting erythromycin or its propanoate with acetylsalicylic acid or DL-alpha-mercaptopropionyl-glycine, in an organic solvent at a temperature between 0° and 30°C, and recovering said compounds.

9. A process according to claim 8, wherein said organic solvent is ethyl acetate.

10. A process for the preparation of a diastereomeric form of a salt of erythromycin or propionylerythromycin with D,L-alpha-mercaptopropionyl-glycine comprising reacting one mole of erythromycin or the propanoate thereof in organic solvents with two moles of D,L-alpha mercaptopropionylglycine, separating a crystalline product consisting of the (+)diastereomeric form; adding another mole of base to the evaporated mother liquors, and separating a crystalline product consisting of the (−)diastereomeric form.

11. A process according to claim 10, wherein erythromycin or the propanoate thereof is dissolved in ethyl acetate and alpha-mercaptopropionylglycine is dissolved in 1-propanol.

12. Pharmaceutical formulations containing a therapeutically effective amount of a compound as claimed in claim 1 as the active ingredient together with pharmaceutically acceptable excipients.

13. Pharmaceutical preparations having antibacterial and antipyretic, anti-inflammatory and antirheumatic activity, characterised by containing a salt of erythromycin or propionylerythromycin with acetylsalicylic acid as the active ingredient.

14. Pharmaceutical preparations having antibacterial, mucolytic and hepatoprotective activity, characterised by containing a salt of erythromycin or propionylerythromycin with alpha-mercaptopropionylglycine as an active ingredient.

15. Use of the salt of erythromycin or propionylerythromycin with acetylsalicylic acid for the manufacture of a medicament for an antipyretic, anti-inflammatory and anti-rheumatic therapeutic application.

16. Use of the salt of erythromycin or propionylerythromycin with alpha-mercaptopropionylglycine for the manufacture of a medicament for an antibacterial, mucolytic and hepatoprotective therapeutic application.

**Patentansprüche**

1. Erythromycin A Derivat der allgemeinen Formel $[X^+ Y^-]$, wobei $X^+$ ein Kation ist des Makrolid-Antibiotikums der Formel:

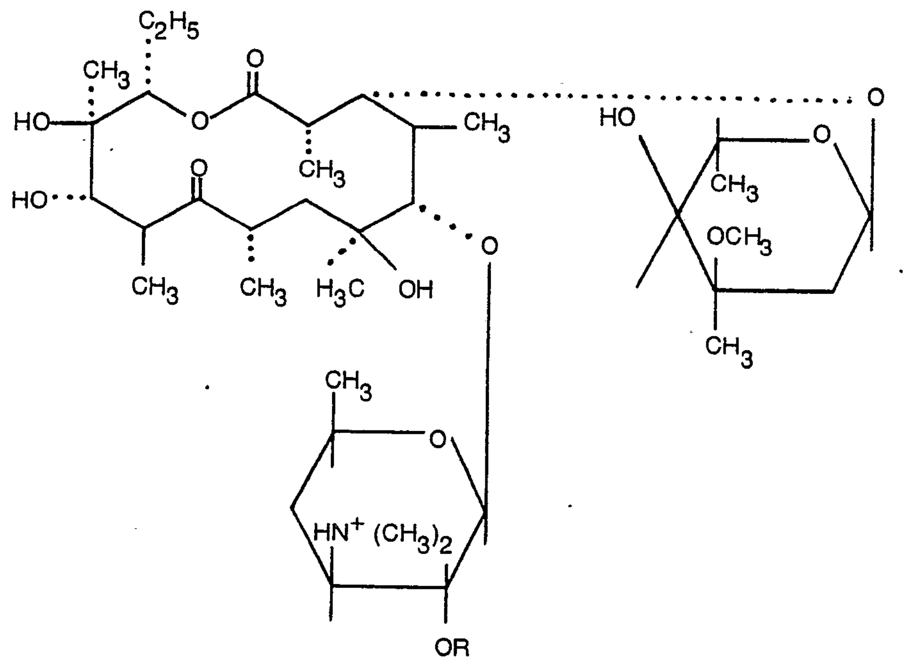

wobei R H oder $—CO—CH_2CH_3$ ist und $Y^-$ das Anion ist von

A) Azetylsalizylsäure der Formel:

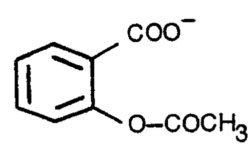

oder

B) DL-alpha-merkaptopropionyl-glyzin oder einer davon enantiomeren Formen der Formel:

$CH_3—CH—CO—NH—CH_2—COO^-$
|
SH

2. Azetylsalizylsäure-Erythromycin Salz gemäss Ansprüch 1.

3. Azetylsalizylsäure-Propionylerthyromycin Salz gemäss Anspruch 1.

4. DL-Alpha-merkaptopropionyl-glyzin-Erythromycin Dalz gemäss Anspruch 1.

5. DL-Alpha-merkaptopropionyl-glyzin-Propionylerythromycin Salz gemäss Anspruch 1.

6. Eine aus einer der beiden enantiomeren Formen des Alpha-merkaptopropionyl-glyzins derivierte diastereomere Form des gemäss Anspruch 4 Alpha-merkaptopropionylglyzin-Erythromycin Salzes.

7. Eine aus einer der beiden enantiomeren Formen des Alpha-merkaptopropionyl-glyzins derivierte diastereoisomere Form des gemäss Anspruch 5 Alpha-merkaptopropionyl-glyzin-Erythoycin Salzes.

8. Verfahren sur Herstellung einer Verbindung gemäss Ansprüchen 1 bis 5, wobei man Erythromycin oder dessen Propanoat mit Azetylsalizylsäure oder DL-Alphamerkaptopropionyl-glyzin, in einem organischen Lösungsmittel, bei einer Temperatur von 0 bis 30°C, umsetzt und die Verbindung erhält.

9. Verfahren gemäss Anspruch 8, wobei das organische Lösungsmittel Ethylazetat ist.

10. Verfahren zur herstellung von einer diastereoisomeren Form eines D,L-Alpha-merkaptopropionyl-glyzin-Erythromycin oder -Propionylerythromycin Salzes, wobei man ein Mol Erythromycin oder dessen Propanoat mit zwei Molen D,L-Alpha-merkaptopropionylglyzin in organischen Lösungsmitteln umsetzt, ein aus der (+)diastereomeren Form bestehendes kristallartiges Produkt trennt, ein zweites Mol dei Base zu der eingedampften Mutterlauge zusetzt und ein aus der (−)diastereoisomeren Form bestehendes Produkt erhält.

11. Verfahren gemäss Anspruch 10, wobei Erythromycin oder dessen Propanoat in Ethylazetat und Alphamerkaptopropionylglyzin in 1-propanol gelöst wird.

12. Pharmazeutische Präparate welche als aktiven Bestandteil eine therapeutisch wirksame Menge von einer der in Anspruch 1 angesprochenen Verbindungen sowie pharmazeutisch verträgliche Arzneiträger enthalten.

13. Pharmazeutische Präparate mit antibakterieller, antipyretischer, entzündungshemmender und antirheumatischer Aktivität, dadurch gekennzeichnet dass sie als aktiven Bestandteil ein Azetylsalizylsäure-Erythromycin oder -Propionylerythromycin Salz enthalten.

14. Pharmazeutische Präparate mit antibakterieller, mukolytischer und leberschützender Aktivität dadurch gekennzeichnet dass sie als aktiven Bestandteil ein Alphamerkaptopropionylglyzin-Erythromycin oder -Propionylerythromycin Salz enthalten.

15. Verwendung von Azetylsalizylsäure-Erythromycin oder -Propionylerythromycin Salz zur Herstellung eines Arzneimittels für antipyretische, entzündungshemmende und antirheumatische therapeutische Anwendung.

16. Verwendung von Alpha-merkaptopropionylglyzin-Erythromycin oder -Propionylerythromycin Salz zur Herstellung eines Arzneimittels für antibakterielle, mucolytische und leberschützende therapeutische Anwendung.

**Revendications**

1. Dérivés d'érythromycine A ayant la formule générale [$X^+$ $Y^-$], où X+ est un cation de l'antibiotique macrolide de formule:

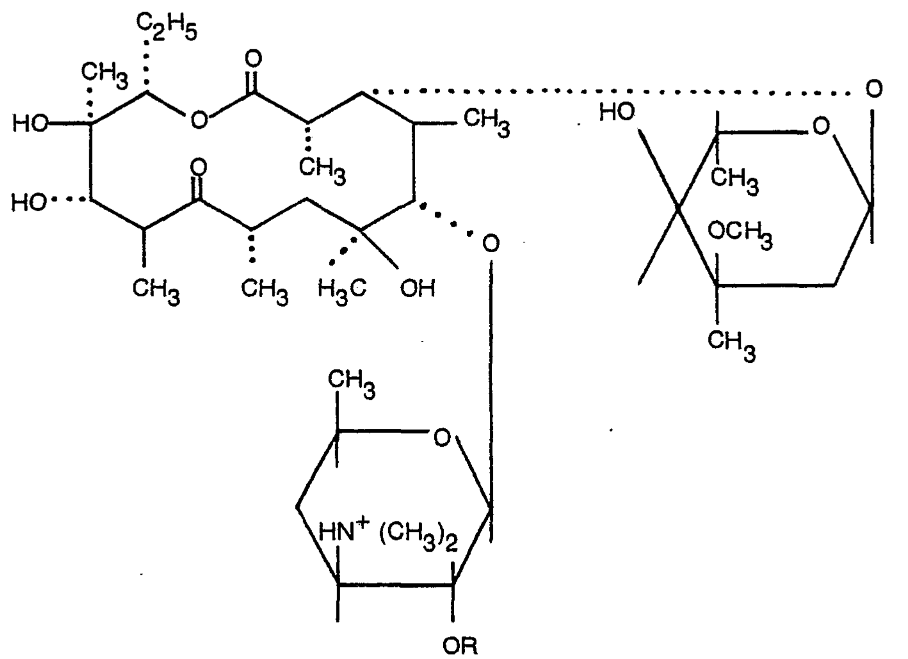

où R est H ou $—CO—CH_2—CH_3$ et $Y^-$ est l'anion de

A) Acide acétylsalicylique de formule

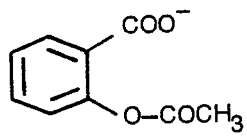

ou

B) DL-alpha-mercaptopropionylglycine ou l'une de ses deux formes énantiomériques de formule:

$$\begin{array}{l} CH_3—CH—CO—NH—CH_2—COO^- \\ \quad\;\;\;\; | \\ \quad\;\;\; SH \end{array}$$

2. Sel d'érythromycine avec de l'acide acétylsalicylique, selon la revendication 1.

3. Sel de propionylérythromycine avec de l'acide acétylsalicylique, selon la revendication 1.

4. Sel d'érythromycine avec DL-alpha-mercaptoprionylglycine, selon la revendication 1.

5. Sel de propionylérythromycine avec DL-alpha-mercaptopropionylglycine selon la revendication 1.

6. Une forme diastéréomérique de sel d'érythromycine avec de l'alpha-mercaptopropionylglycine, selon la revendication 4, dérivé de l'une des deux formes énantiomériques de alpha-mercaptopropionylglycine.

7. Une forme diastéréomérique de sel de propionylérythromycine avec alpha-mercaptopropionylglycine, selon la revendication 5, dérivé de l'une des deux formes énantiomériques de alpha-mercaptopropionylglycine.

8. Un procédé pour la préparation des composants ainsi que revendiqués dans les revendications 1 à 5 où l'on fait réagir de l'érythromycine ou son propanoate avec de l'acide acétylsalicylique ou du DL-alpha-mercaptopropionylglycine, dans un solvent organique à une température entre 0° et 30°C, et récupérant les dits composants.

9. Un procédé selon la revendication 8, où le dit solvent organique est de l'acétate d'éthyl.

10. Un procédé pour la préparation d'une forme diastéréomérique d'un sel d'érythromycine ou de propionyléthromycine avec DL-alpha-mercaptopropionylglycine, où l'on fait réagir une mole d'érythromycine ou le propanoate de celle-ci dans des solvents organiques avec deux moles de DL-alpha-mercaptopropionylglycine, on sépare un produit cristallisé consistant dans la forme diastéréomérique (+); on ajoute une autre mole de base au liqueur mère évaporé et on sépare un produit cristallisé consistant dans la forme diastéréomérique (−).

11. Un procédé selon la revendication 10, où l'érythromycine ou son propanoate est dissout dans de l'acétate d'éthyl et l'alpha-mercaptopropionylglycine et dissout dans 1-propanol.

12. Formulation pharmaceutique contenant une quantité thérapeutiquement efficace d'une composition telle que revendiquée dans la revendication 1 comme ingrédient actif, avec des excipients pharmaceutiquement acceptables.

13. Préparation pharmaceutique ayant une activité bactéricide et antipyrétique, antiinflammatoire et antirheumatismale, caractérisé par le fait de contenir un sel d'érythromycine ou de propionylérythromycine avec de l'acide acétylsalicylique comme ingrédient actif.

14. Préparation pharmaceutique ayant une activité bactéricide, mucolytique et hépatoprotective, caractérisée par le fait de contenir un sel d'érythromycine ou de propionyl érythromycine avec de l'alpha-mercaptopropionylglycine comme ingrédient actif.

15. Utilisation du sel d'érythromycine ou de propionylérythromycine avec de l'acide acétylsalicylique pour la fabrication d'un médicament pour l'application thérapeutique à effet antipyrétique, antiinflammatoire et antirheumatismale.

16. Utilisation d'un sel d'érythromycine ou de propionylérythromycine avec de l'alpha-mercaptopropionylglycine pour la fabrication d'un médicament pour application thérapeutique bactéricide, mucolytique et hépatoprotective.